# EUROPEAN PATENT APPLICATION

(11) **EP 0 852 141 A1**
(43) Date of publication of application: **08.07.1998**
(21) Application number: 96113165.3
(22) Date of filing: 16.08.1996
(51) Int. Cl.: A61K 9/14, A61K 31/44

(54) **Nifedipine containing pharmaceutical extended release composition and a process for the preparation thereof**

(71) Applicant: J.B. Chemicals & Pharmaceuticals Ltd., Mumbai 400 025, Maharashtra (IN)
(72) Inventor: Mody, Shirish Bhagwanlal, Mumbai - 400 006, Maharashtra (IN); Doshi, Madhukant Mansukhlal, Dr., Mumbai - 400 077, Maharashtra (IN); Joshi, Milind Dattaraya, Dr., Thane - 400 604, Maharashtra (IN)
(74) Representative: Tergau, Enno, Dipl.-Ing.

(57) **Abstract**

A pharmaceutical extended release composition comprises of co-precipitates of Nifedipine and Polyvinylpyrolodine embedded in a pharmaceutically acceptable diluent such as Lactose and Hydrophillic Matrix forming co-polymer such as hydroxypropylmethylcellulose produced by a novel process. Solid dosage form prepared with the mixture thus obtained will serve a extended release effect that a single daily administration can be effected whilst maintaining bio-availability similar to other products available in the market .

## Description

### Field of the invention

The present invention relates to the preparation of Nifedipine containing pharmaceutical extended release composition and a process for the preparation thereof. In particular the present invention relates to pharmaceutical formulations which provide controlled release at regular rates and within desired periods of the pharmacologically useful and active drug 'Nifedipine', among others, for Vasopastic Angina, chronic stable Angina (Classical effort Associated Angina) and hypertension.

### Background of the Prior Art.

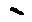 Nifedipine' is a drug belonging to the class of pharmacological agents known as the calcium channel blockers belonging to the group of 1,4-dihydropyridines. Nifedipine is practically insoluable in water and hence co-precipitates with certain known polymer with high molecular weight such as polyethylene glycol or an ester or ether of polyethylene glycol or with other selected materials, including polyvinylpyrrolidone.
Nifedipine is a peripheral arterial vasodilator which acts directly on vascular smooth muscle. The binding of Nifedipine to voltage-dependent and possible receptor-operated channels in vascular smooth muscle are limited and thus dependent upon the influx of extracellular calcium for contraction to occur. The reduction in calcium influx by Nifedipine causes arterial, vasodilation and decreased peripheral vascular resistance which results in reduced arterial blood pressure.

Nifedipine is available in a number of formulations. Liquid - filled capsules with a relatively rapid onset but short duration of action are administered three times daily, they have sometimes been used for buccal or sublingual administration. There are also tablets and capsules with a slower onset and longer duration of action, enabling twice daily administration, although these are often referred to by nomenclature implying extended or sustained release, they should be distinguished from the true extended - release preparations available in some countries that allow administration once daily. Those skilled in the art will appreciate that for the treatment of hypertension, it is more desirable to maintain plasma nifedipine concentrations within a therapeutic window of about 20-80 ng/ml, and it is desirable to have once a day slow release preparations of the substance .

The references given below are that of some of the once a day Nifedipine extended release preperations.

United States Patent No. 5,145,683 describes a pharmaceutical composition which comprises particles of a finely divided pharmaceutically acceptable water soluble diluent coated with microcrystalline particles of Nifedipine, the majority of which have a particle size of 100 micrometers or less, in the presence of polyvinylpyrrolidone, the polyvinylpyrrolidone being present in an amount of from 10 to 90% by weight based on the weight of Nifedipine.

United States Patent No. 4,973,469 describes a controlled release formulation of Nifedipine comprising an adsorbate of a mixture of a pharmaceutically useful active ingredient and an inactive substance adsorbed on a cross-linked polymer. The inactive substance is selected to modify the dissolution of the active ingredient from the cross-linked polymer in vivo.

Another such slow release formulation in the form of tablet produced by Bayer AG. Germany is reported in the Drug Invest 5(5):243-249, 1993 . The tablet consists of a core, and a coat that is pressed around the core. The core contains part of the nifedipine dose in an immediate release formulation, while the coat contains nifedipine in a modified release formulation. On contact with water the surface of the tablet begins to swell and there is gradual erosion of the external tablet coat. After complete erosion of the external coat, the nifedipine in the core is rapidly released. This principle permits a constant release of nifedipine for approximately 12 hours followed by a terminal release of the drug towards the end of a 16-hour period.

Another slow release formulation in the form of tablet, 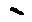 Procardia XL' is invented by Alza Corpn. and marketed by Pfizer Inc. Procardia XL preparation is based on osmotic push-pull principle. (Physicians Desk Reference, 4th Edition p.1908).The tablet is similar in appearance to a conventional tablet. It consists, however, of a semipermeable membrane surrounding an osmotically active drug core. The core itself is divided into two layers: an "active" layer containing the drug, and a "push" layer containing pharmacologically inert (but osmotically active) components. As water from the gastrointestinal tract enters the tablet, pressure increases in the osmotic layer and "pushes" against the drug layer, releasing drug through the precision laser-drilled tablet orifice in the active layer.One of the main problem arising from push-pull osmotic technology is that it requires specialised technology for drilling the orifice on the surface of the tablet due to which it requires high precision equipments. Therefore, it is costly and also time consuming.

The formulation obtained by the present invention is more economical compared to other once a day Nifedipine preparations already in the market. The inventors of the present invention have come out with a simple and inexpensive way of producing once a day extended release Nifedipine tablets.

### Objects of the Invention:-

It is an object of the invention to provide an oral formulation of Nifedipine which exhibits sustained or controlled characteristics. It is also an object of the invention to provide a oral formulation with a novel drug absorption technology designed specifically to improve the solubility and absorption characteristics of poorly water soluble drugs like Nifedipine. Such compounds tend to suffer from sub-optimal absorption and, as a result rehire frequent oral administration. This novel technology enhances the solubility of the drug and controls its delivery over a prolonged predetermined period for 24 hours. The formulation technology of the present invention allows the smooth and continous absorption, eliminating the wide peak through pharmacokinetic fluctuations.

These and other objects of the present invention may be readily gleened from a reading of the description of the invention given below.

### BRIEF DESCRIPTION OF THE INVENTION

The present invention provides the preparation of Nifedipine containing pharmaceutical extended release composition and a process for the preparation thereof. The formulation prepared by the present invention shows prolonged and nearly constant rate of drug absorption for a long period of time for 24 hours and concurrently maintains a high degree of bioavailability.

In vivo trials have shown that the active agent is released at a controlled rate over long periods which can be extended upto 24 hours.

The present invention process comprises of co-precipitating Nifedipine with Polyvinylpyrrolidone on a mixture of pharmaceutically acceptable water soluble excipient such as lactose and a hydrophillic matrix forming polymer such as hydroxypropylmethylcellulose by dissolving Nifedipine & Polyvinylpyrrolidone in a solvent mixture. The solvent mixtures required to be used in the process are of the type that Nifedipine, Polyvinylpyrrolidone and gel forming polymer are all soluble in the mixture. The solvent mixtures are suitably selected from Methyl Alcohol, Isopropyl Alcohol, Ethyl Alcohol and Chlorinated Hydrocarbon like Chloroform, Methylene chloride, and Carbon tetrachloride. Specially preferred solvents include a mixture of Isopropyl alcohol and methylene chloride The solution is adsorbed on the mixture of lactose and hydroxypropylmethylcellulose,the wet mass obtained is dried in an oven to remove the solvents. The resulting mixture is then granulated, blended with a lubricant like magnesium stearate and tabletted and coated as per the commonly used pharmaceutical technology.

Average Molecular weight of Polyvinylpyrrolidone(K 30 grade) utilised is about 40,000 and ratio of Nifedipine to Polyvinylpyrrolidone is preferably between 1:1 to 1:3. Ratio of Nifedipine to pharmacetically acceptable water soluble excipient-lactose varies between 1:3 to 1:10 and ratio of Nifedipine to Hydrophillic matrix forming polymer Hydroxypropylmethylcellulose ranges between 1:0.5 to 1:3. Various grades of Hydroxypropylmethylcellulose ranging from 4000 cps to 100,000 cps. can be utilized. Lactose can also be replaced with other sugars like glucose, sucrose, mannitol, galactose, and the like. Hydrophillic matrix forming polymer Hydroxypropylmethylcellulose can be replaced by other polymers or a mixture of polymers such as methylcellulose, Hydroxyethylcellulose and the like.
Due to utilization of such solvent mixtures in which Nifedipine, Polyvinylpyrrolidone & Hydrophillic gel forming polymer (Hydroxypropylmethylcellulose) are soluble, it is during adsorption of the solution of Nifedipine & Polyvinylpyrrolidone on the mixture of lactose & Hydroxypropylmethylcellulose, the co-precipitates of Nifedipine & Polyvinylpyrrolidone get embedded in such a way that the adsorbate results in a prolonged extended release of Nifedipine from the solid composition like tablets.

The excipients and solvents required to be used at various stages of the process are selected from pharmaceutically acceptable excipients normally employed in pharmaceutical formulation. Those skilled in the art can decide as to the particular excipients and solvents and quantities thereof to be employed in the process, having regard for the quantity of the active drug to be taken, and rates and periods of release of the active drug desired in the final formulation.
The invention will be more fully understood from the following examples. These examples are to be construed as illustrative of the invention and not limitative thereof :

### Example 1

Polyvinylpyrrolidone (K 30 grade ) (0.3 kg) and Nifedipine (0.3 kg) were dissolved in a solvent mixture consisting of Isopropyl Alcohol (0.6 kg) and Methylene chloride (1.2 kg). The solution was then adsorbed on a mixture of lactose (1.6 kg) and Hydroxypropylmethylcellulose (K15 M grade) (0.4 kg), thereafter the solvent was evaporated in the tray drying oven from the wet mass. The resulting dried mass was then passed through a 2 mm sieve on a multimill to obtain a granulation. The granules thus obtained were blended with Magnesium Stearate (0.075 kg) and tabletted to obtain tablet containing 30 mg of Nifedipine. Tablets were then coated with characteristic cosmetic colour coating for identification.

### Example 2

The procedure employed was similar to that in Example 1, except that the amount of Hydroxypropylmethylcellulose (K 15 M grade) was decreased to 0.3 kg and Hydroxypropylmethylcellulose (K 100 M grade) was added at a level of 0.1 kg.

### Example 3

The procedure employed was similar to that in Example 1, except that Lactose content was decreased from 1.6 kg to 1.4 kg and Mannitol was included in the amount of 0.2 kg.

### Example 4

The procedure employed was similar to that in Example 1, except that Methylene chloride was replaced by equivalent quantity of chloroform 1.2 kg.

### Example 5

The procedure employed was similar to that in Example 1, except that Isopropyl Alcohol was replaced by equivalent amount of methyl alcohol 0.6 kg.

### Example 6

The procedure employed was similar to that in examples 1 to 5 herein above, except that 60 mg of Nifedipine per tablet was used instead of 30 mg of Nifedipine per tablet and the excipients being used were taken in appropriate quantity as given in the aforesaid examples by keeping the ratio of active drug to the excipients constant.

### Example 7

The procedure employed was similar to that in examples 1 to 5 herein above, except that 90 mg of Nifedipine per tablet was used instead of 30 mg of Nifedipine per tablet and the excipients being used were taken in appropriate quantity as given in the aforesaid examples by keeping the ratio of active drug to the excipients constant.

It is to be understood that the examples and embodiments described hereinabove are for the purposes of providing a description of the present invention by way of examples and are not to be viewed as limiting the present invention in any way. Various modifications or changes that may be made to that described hereinabove by those of ordinary skill in the art are also contemplated by the present invention and are to be included within the spirit and purview of this application and the appended claims.

### DISSOLUTION STUDIES :

1. Dissolution studies were carried as per U.S.P. in paddle type apparatus.
   Nifedipine extended release tablets 30 mg prepared as per Example 1 hereinabove, were tested against the 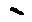 Procardia XL' 30 mg. The comparative dissolution data indicates comparable dissolution profile for both.
2. Nifedipine extended release tablets 60 mg prepared as per Example 6 hereinabove, were tested against the 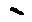 Procardia XL' 30 mg. The comparative dissolution data indicates comparable dissolution profile for both.
3. Bioequivalence studies
   Nifedipine extended release tablets 30 mg as prepared by example 1 hereinabove, were subjected to Bioequivalence studies on 12 volunteers (crossover design) with 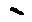 Procardia XL' 30 mg and the results are presented as under :
Comparative Bioavailability of Nifedipine Tablets 30 mg (Example 1) & Procardia XL Tablets, 30 mg (Pratt) is given below:
Mean volunteer plasma concentration (ng/ml)

| EXAMPLE 1 | | PROCARDIA XL. | |
|---|---|---|---|
| Time (Hrs) | Mean Plasma Conc.in ng/ml | Time(hrs) | Mean Plasma Conc.in ng/ml |
| 0 | 0 | 0 | 0 |
| 0.5 | 7 | 0.5 | 9 |
| 1 | 12 | 1 | 14 |
| 2 | 24.3 | 2 | 21.7 |
| 2.5 | 30 | 2.5 | 35.7 |
| 3 | 41.8 | 3 | 42.6 |
| 4 | 52.6 | 4 | 59.0 |
| 6 | 50.5 | 6 | 64.2 |
| 8 | 43.0 | 8 | 47.3 |
| 10 | 37.7 | 10 | 36.0 |
| 12 | 31.0 | 12 | 26.0 |
| 14 | 28.0 | 14 | 21.2 |
| 18 | 12.0 | 18 | 10.4 |
| 24 | 4.6 | 24 | 5.9 |
| 48 | 0 | 48 | 0 |

### Pharmacokinetic Parameters:

| Parameters | Nifedipine tablets as prepared under example 1 | Procardia XL |
|---|---|---|
| C Max(ng/ml) | 52.74 ± 0.58 | 53.96 ± 0.2 |
| T Max(hrs) | 4.0 ± 0.0 | 4.0 ± 0.0 |
| AUC ng/ml.hr | 88.47 ± 15.20 | 87.19 ± 10.00 |

The results of the above studies indicate that Example 1 product is comparable to the reference product in bioavailability.
3. The tablets prepared as per Examples 2 to 5 exhibited similar in vitro dissolution rate as that of example 1.
4. The tablets prepared as per Examples 6 and 7 hereinabove also exhibited satisfactory results of in vitro dissolution profiles.

## Claims

1. A Pharmaceutical extended release composition containing Nifedipine as an active ingredient characterising by the fact that the active substance is co-precipitated in situ with polyvinylpyrrolidone, the co-precipitate thus formed are embedded in a mixture of pharmaceutically acceptable water soluble excipient such as lactose and a hydrophillic matrix forming polymer such as Hydroxymethylcellulose, said composition providing prolonged and nearly constant rate of drug absorption for a extended period of at least about 24 hours with high degree of bio availability.

2. A Pharmaceutical composition according to claim 1. wherein 1the ratio between Nifedipine and polyvinylpyrrolidone varies between 1:1 to 1:3

3. A Pharmaceutical composition according to claim 1, wherein the average molecular weight of polyvinylpyrrolidone is approximately 40,000.

4. A Pharmaceutical composition according to claim 1, wherein the ratio of Nifedipine to lactose is between 1:3 to 1:10

5. A Pharmaceutical composition according to claim 1, wherein various grades of the Hydroxypropylmethylcellulose ranging from 4000 cps to 100,000 cps can be used .

6. A Pharmaceutical composition according to claim 1 wherein the ratio of Nifedipine to Hydroxypropylmethylcellulose varies between 1:0.5 to 1:3.

7. A Pharmaceutical composition according to claim 1, wherein lactose can be replaced by other inert excipients like mannitol, sorbitol, sucrose, glucose and the like.

8. A Pharmaceutical composition according to claim 1, wherein Hydroxypropylmethylcellulose can be replaced by other polymers such as Hydroxyethylcellulose, methylcellulose, and the like.

9. A Pharmaceutical composition according to claim 1, in the form of extended release tablets.

10. A Pharmaceutical composition according to claim 1, in the form of tablets characterised by the fact that it has more satisfying retard effect and that a single daily administration can be effective whilst maintaining the bioavailability and the therapeutic efficacy unaltered with respect to conventional products.

11. A form of oral administration based on Nifedipine containing solid pharmaceutical formulation as specified in claim 1.

12. The formulation according to claim 1, containing about 30 mg, or 60 mg or 90 mg of Nifedipine per tablet.

13. Process for the preparation of extended release pharmaceutical composition according to claim 1, comprising of co-precipitating Nifedipine with Polyvinylpyrrolidone on a mixture of pharmaceutically acceptable water soluble excipient such as lactose and a hydrophillic matrix forming polymer such as hydroxypropylmethylcellulose by dissolving Nifedipine & Polyvinylpyrrolidone in a solvent mixture, the resultant solution is adsorbed on a mixture of lactose and hydroxypropylmethylcellulose,the wet mass obtained is dried in an oven to remove the solvents, the resulting mixture is then granulated, blended with a lubricant like magnesium stearate, tabletted and coated as per the commonly used pharmaceutical technology.

14. Process according to claim 13, wherein said solvent mixtures are suitably selected from Methyl Alcohol, Isopropyl Alcohol, Ethyl Alcohol or Chlorinated Hydrocarbon like Chloroform,Methylene chloride, and Carbon tetrachloride, specially preferred solvents include a mixture of Isopropyl alcohol and methylene chloride.

15. Process according to claim 13, wherein ratio of Nifedipine to polyvinylpyrrolidone is between 1:1 to 1:3

16. Process according to claim 13, wherein average molecular weight of polyvinylpyrrolidone is about 40,000 (Polyvinylpyrrolidone K30 grade)

17. Process according to claim 13, wherein inert excepient lactose is replaced preferably by sucrose, mannitol, sorbitol, glucose and the like.

18. Process according to claim 13, wherein Hydrophillic matrix forming polymer Hydroxypropylmethylcellulose is replaced by methylcellulose, Hydroxyetylcellulose, and the like.

19. Process according to claim 13, wherein ratio between Nifedipine and inert excipient varies between 1:3 to 1:10

20. Process according to claim 13, wherein ratio between Nifedipine & Hydrophyllic matrix forming polymer is between 1:0.5 to 1:3.

21. Process according to claim 13, wherein solid pharmaceutical product is tablet.

22. Process according to claim 14, wherein Nifedipine used is 30mg or 60mg or 90 mg per tablet substantially as herein described in the foregoing examples.

23. (3) A pharmaceutical composition according to claim 2, wherein the average molecular weight of polyvinylpyrrolidone is approximately 40,000.

24. (9) A pharmaceutical composition according to claim 2, in the form of extended release tablets.

25. (9) A pharmaceutical composition according to claim 3, in the form of extended release tablets.

26. (9) A pharmaceutical composition according to claim 4, in the form of extended release tablets.

27. (9) A pharmaceutical composition according to claim 5, in the form of extended release tablets.

28. (9) A pharmaceutical composition according to claim 6, in the form of extended release tablets.

29. (9) A pharmaceutical composition according to claim 7, in the form of extended release tablets.

30. (9) A pharmaceutical composition according to claim 8, in the form of extended release tablets.

31. (10) A Pharmaceutical composition according to claim 2, in the form of tablets characterised by the fact that it has more satisfying retard effect and that a single daily administration can be effective whilst maintaining the bioavailability and the therapeutic efficacy unaltered with respect to conventional products.

32. (10) A Pharmaceutical composition according to claim 3, in the form of tablets characterised by the fact that it has more satisfying retard effect and that a single daily administration can be effective whilst maintaining the bioavailability and the therapeutic efficacy unaltered with respect to conventional products.

33. (10) A Pharmaceutical composition according to claim 4, in the form of tablets characterised by the fact that it has more satisfying retard effect and that a single daily administration can be effective whilst maintaining the bioavailability and the therapeutic efficacy unaltered with respect to conventional products.

34. (10) A Pharmaceutical composition according to claim 5, in the form of tablets characterised by the fact that it has more satisfying retard effect and that a single daily administration can be effective whilst maintaining the bioavailability and the therapeutic efficacy unaltered with respect to conventional products.

35. (10) A Pharmaceutical composition according to claim 6, in the form of tablets characterised by the fact that it has more satisfying retard effect and that a single daily administration can be effective whilst maintaining the bioavailability and the therapeutic efficacy unaltered with respect to conventional products.

36. (10) A Pharmaceutical composition according to claim 7, in the form of tablets characterised by the fact that it has more satisfying retard effect and that a single daily administration can be effective whilst maintaining the bioavailability and the therapeutic efficacy unaltered with respect to conventional products.

37. (10) A Pharmaceutical composition according to claim 8, in the form of tablets characterised by the fact that it has more satisfying retard effect and that a single daily administration can be effective whilst maintaining the bioavailability and the therapeutic efficacy unaltered with respect to conventional products.

38. (10) A Pharmaceutical composition according to claim 9, in the form of tablets characterised by the fact that it has more satisfying retard effect and that a single daily administration can be effective whilst maintaining the bioavailability and the therapeutic efficacy unaltered with respect to conventional products.

39. (10) A Pharmaceutical composition according to claim 23(3), in the form of tablets characterised by the fact that it has more satisfying retard effect and that a single daily administration can be effective whilst maintaining the bioavailability and the therapeutic efficacy unaltered with respect to conventional products.

40. (10) A Pharmaceutical composition according to claim 24(9), in the form of tablets characterised by the fact that it has more satisfying retard effect and that a single daily administration can be effective whilst maintaining the bioavailability and the therapeutic efficacy unaltered with respect to conventional products.

41. (10) A Pharmaceutical composition according to claim 25(9), in the form of tablets characterised by the fact that it has more satisfying retard effect and that a single daily administration can be effective whilst maintaining the bioavailability and the therapeutic efficacy unaltered with respect to conventional products.

42. (10) A Pharmaceutical composition according to claim 26(9), in the form of tablets characterised by the fact that it has more satisfying retard effect and that a single daily administration can be effective whilst maintaining the bioavailability and the therapeutic efficacy unaltered with respect to conventional products.

43. (10) A Pharmaceutical composition according to claim 27(9), in the form of tablets characterised by the fact that it has more satisfying retard effect and that a single daily administration can be effective whilst maintaining the bioavailability and the therapeutic efficacy unaltered with respect to conventional products.

44. (10) A Pharmaceutical composition according to claim 28(9), in the form of tablets characterised by the fact that it has more satisfying retard effect and that a single daily administration can be effective whilst maintaining the bioavailability and the therapeutic efficacy unaltered with respect to conventional products.

45. (10) A Pharmaceutical composition according to claim 29(9), in the form of tablets characterised by the fact that it has more satisfying retard effect and that a single daily administration can be effective whilst maintaining the bioavailability and the therapeutic efficacy unaltered with respect to conventional products.

46. (10) A Pharmaceutical composition according to claim 30(9), in the form of tablets characterised by the fact that it has more satisfying retard effect and that a single daily administration can be effective whilst maintaining the bioavailability and the therapeutic efficacy unaltered with respect to conventional products.

47. (11) A form of oral administration based on Nifedipine con taining solid pharmaceutical formulation as specified in claim 2.

48. (11) A form of oral administration based on Nifedipine con taining solid pharmaceutical formulation as specified in claim 3.

49. (11) A form of oral administration based on Nifedipine containing solid pharmaceutical formulation as specified in claim 4.

50. (11) A form of oral administration based on Nifedipine con taining solid pharmaceutical formulation as specified in claim 5.

51. (11) A form of oral administration based on Nifedipine con taining solid pharmaceutical formulation as specified in claim 6.

52. (11) A form of oral administration based on Nifedipine con taining solid pharmaceutical formulation as specified in claim 7.

53. (11) A form of oral administration based on Nifedipine containing solid pharmaceutical formulation as specified in claim 8.

54. (11) A form of oral administration based on Nifedipine con taining solid pharmaceutical formulation as specified in claim 9.

55. (11) A form of oral administration based on Nifedipine con taining solid pharmaceutical formulation as specified in claim 10.

56. (11) A form of oral administration based on Nifedipine con taining solid pharmaceutical formulation as specified in claim 23 (3).

57. (11) A form of oral administration based on Nifedipine containing solid pharmaceutical formulation as specified in claim 24 (9).

58. (11) A form of oral administration based on Nifedipine containing solid pharmaceutical formulation as specified in claim 25 (9).

59. (11) A form of oral administration based on Nifedipine containing solid pharmaceutical formulation as specified in claim 26 (9).

60. (11) A form of oral administration based on Nifedipine containing solid pharmaceutical formulation as specified in claim 27 (9).

61. (11) A form of oral administration based on Nifedipine containing solid pharmaceutical formulation as specified in claim 28 (9).

62. (11) A form of oral administration based on Nifedipine containing solid pharmaceutical formulation as specified in claim 29 (9).

63. (11) A form of oral administration based on Nifedipine containing solid pharmaceutical formulation as specified in claim 30 (9).

64. (11) A form of oral administration based on Nifedipine containing solid pharmaceutical formulation as specified in claim 31 (10).

65. (11) A form of oral administration based on Nifedipine containing solid pharmaceutical formulation as specified in claim 32 (10).

66. (11) A form of oral administration based on Nifedipine containing solid pharmaceutical formulation as specified in claim 33 (10).

67. (11) A form of oral administration based on Nifedipine containing solid pharmaceutical formulation as specified in claim 34 (10).

68. (11) A form of oral administration based on Nifedipine containing solid pharmaceutical formulation as specified in claim 35 (10).

69. (11) A form of oral administration based on Nifedipine containing solid pharmaceutical formulation as specified in claim 36 (10).

70. (11) A form of oral administration based on Nifedipine containing solid pharmaceutical formulation as specified in claim 37 (10).

71. (11) A form of oral administration based on Nifedipine containing solid pharmaceutical formulation as specified in claim 38 (10).

72. (11) A form of oral administration based on Nifedipine containing solid pharmaceutical formulation as specified in claim 39 (10).

73. (11) A form of oral administration based on Nifedipine containing solid pharmaceutical formulation as specified in claim 40 (10).

74. (11) A form of oral administration based on Nifedipine containing solid pharmaceutical formulation as specified in claim 41 (10).

75. (11) A form of oral administration based on Nifedipine containing solid pharmaceutical formulation as specified in claim 42 (10).

76. (11) A form of oral administration based on Nifedipine containing solid pharmaceutical formulation as specified in claim 43 (10).

77. (11) A form of oral administration based on Nifedipine containing solid pharmaceutical formulation as specified in claim 44 (10).

78. (11) A form of oral administration based on Nifedipine containing solid pharmaceutical formulation as specified in claim 45 (10).

79. (11) A form of oral administration based on Nifedipine containing solid pharmaceutical formulation as specified in claim 46 (10).
